(19) Europäisches Patentamt
European Patent Office
Office européen des brevets

(11) Veröffentlichungsnummer: **0 574 774 A1**

# (12) EUROPÄISCHE PATENTANMELDUNG

(21) Anmeldenummer: **93108996.5**

(22) Anmeldetag: **04.06.93**

(51) Int. Cl.5: **C07D 277/64**, C07D 215/14, A61K 31/425, A61K 31/495

(30) Priorität: **17.06.92 DE 4219765**

(43) Veröffentlichungstag der Anmeldung: **22.12.93 Patentblatt 93/51**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Mohrs, Klaus-Helmut, Dr.**
**Wildsteig 24**
**W-5600 Wuppertal 1(DE)**
Erfinder: **Raddatz, Siegfried, Dr.**
**Jakob-Böhme-Strasse 21**
**W-5000 Köln 80(DE)**
Erfinder: **Matzke, Michael, Dr.**
**Am Ringelbusch 15**
**W-5600 Wuppertal 1(DE)**
Erfinder: **Fugmann, Burkhard, Dr.**
**Gerhardstrasse 47**
**W-4030 Ratingen 1(DE)**
Erfinder: **Fruchtmann, Romanis**
**Weidenpescher Strasse 14**
**W-5000 Köln 60(DE)**
Erfinder: **Hatzelmann, Armin, Dr.**
**Alter Wall 3**
**W-7750 Konstanz(DE)**
Erfinder: **Müller-Peddinghaus, Reiner, Prof. Dr.**
**Klutstein 22a**
**W-5060 Bergisch Gladbach 2(DE)**

## (54) Substituierte (Benzothiazolyl- und Chinoxalyl-methoxy)phenyl-essigsäurederivate.

(57) Substituierte (Benzothiazolyl- und Chinoxalyl-methoxy)phenyl-essigsäurederivate werden hergestellt entweder durch Umsetzung von Heterocyclylmethylhalogen-Verbindungen mit entsprechenden Phenoxyverbindungen, entweder direkt oder mit anschließender Alkylierung, oder durch Umsetzung der Heterocyclylmethylhalogen-Verbindung mit Phenoxyverbindungen und anschließender Umsetzung mit geeigneten Grignard-Verbindungen. Die substituierten (Benzothiazolyl- und Chinoxalyl-methoxy)phenyl-essigsäurederivate können als Wirkstoffe in Arzneimitteln eingesetzt werden.



Rank Xerox (UK) Business Services
(3.10/3.6/3.3.1)

Die Erfindung betrifft substituierte (Benzothiazolyl- und Chinoxalinyl-methoxy)phenyl-essigsäurederivate, Verfahren Zu ihrer Herstellung und ihre Verwendung in Arzneimitteln.

Es ist bereits bekannt, daß α-substituierte 4-(Chinolin-2-yl-methoxy)phenylessigsäurederivate eine lipoxygenaseinhibierende Wirkung besitzen [vgl. EP 344 519 (US 4 970 215) und EP 339 416].

Die vorliegende Erfindung betrifft substituierte (Benzothiazolyl- und Chinoxalyl-methoxy)phenyl-essigsäurederivate der allgemeinen Formel (I)

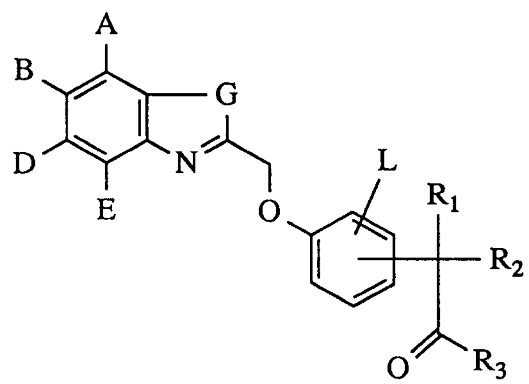

(I),

in welcher

| | |
|---|---|
| A, B, D, E und L | gleich oder verschieden sind und<br>für Wasserstoff, Hydroxy, Halogen, Trifluormethyl, Trifluormethoxy oder Carboxy stehen,<br>für geradkettiges oder verzweigtes Alkyl mit bis zu 10 Kohlenstoffatomen stehen, das gegebenenfalls durch Hydroxy oder Halogen substituiert ist,<br>für geradkettiges oder verzweigtes Alkoxy oder Alkoxycarbonyl mit bis zu 10 Kohlenstoffatomen stehen, oder<br>für Aryl mit 6 bis 10 Kohlenstoffatomen stehen, das gegebenenfalls durch Halogen, Nitro, Cyano oder durch geradkettiges oder verzweigtes Alkyl oder Alkoxy mit jeweils bis zu 8 Kohlenstoffatomen substituiert ist, |
| G | für ein Schwefelatom oder für die Gruppe der Formel -N=CH- steht, |
| $R^1$ | für Cycloalkyl mit 3 bis 12 Kohlenstoffatomen steht, oder<br>für geradkettiges oder verzweigtes Alkyl mit bis zu 10 Kohlenstoffatomen steht, das gegebenenfalls durch Aryl mit 6 bis 10 Kohlenstoffatomen substituiert ist, |
| $R^2$ | für Wasserstoff, oder<br>für eine Gruppe der Formel $-OR^4$ steht,<br>worin |
| $R^4$ | Wasserstoff, geradkettiges oder verzweigtes Alkyl mit bis zu 8 Kohlenstoffatomen, Benzyl oder Phenyl bedeutet, |
| $R^3$ | für Hydroxy, geradkettiges oder verzweigtes Alkoxy mit bis zu 8 Kohlenstoffatomen oder Phenoxy steht, oder<br>für eine Gruppe der Formel $-NHSO_2-R^5$ steht,<br>worin |
| $R^5$ | geradkettiges oder verzweigtes Alkyl mit bis zu 8 Kohlenstoffatomen bedeutet, das gegebenenfalls durch Phenyl substituiert ist, das seinerseits durch geradkettiges oder verzweigtes Alkyl mit bis zu 6 Kohlenstoffatomen oder Halogen substituiert sein kann, oder<br>Phenyl bedeutet, das gegebenenfalls durch Halogen, Trifluormethyl oder durch geradkettiges oder verzweigtes Alkyl oder Alkoxy mit jeweils bis zu 6 Kohlenstoffatomen substituiert sein kann |

und deren Salze.

Im Rahmen der vorliegenden Erfindung werden physiologisch unbedenkliche Salze bevorzugt. Physiologisch unbedenkliche Salze der substituierten (Benzothiazolyl- und Chinoxalyl-methoxy)phenyl-essigsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Metallsalze, bevorzugt der einwertigen Metalle, und die Ammoniumsalze. Bevorzugt werden Alkalisalze wie beispielsweise Natrium-, Kalium- und Ammoniumsalze.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

- A, B, D, E und L gleich oder verschieden sind und
  für Wasserstoff, Fluor, Chlor, Brom, Trifluormethoxy oder Carboxy stehen,
  für geradkettiges oder verzweigtes Alkyl mit bis zu 8 Kohlenstoffatomen stehen, das gegebenenfalls durch Hydroxy, Fluor, Chlor oder Brom substituiert ist,
  für geradkettiges oder verzweigtes Alkoxy oder Alkoxycarbonyl mit jeweils bis zu 8 Kohlenstoffatomen stehen, oder
  für Phenyl stehen, das gegebenenfalls durch Fluor, Chlor, Brom, Nitro, Cyano oder durch geradkettiges oder verzweigtes Alkyl oder Alkoxy mit jeweils bis zu 6 Kohlenstoffatomen substituiert ist,
- G für ein Schwefelatom oder für die Gruppe der Formel -N=CH- steht,
- $R^1$ für Cyclopropyl, Cyclobutyl, Cyclopentyl, Cyclohexyl, Cycloheptyl oder Cyclooctyl steht, oder
  für geradkettiges oder verzweigtes Alkyl mit bis zu 8 Kohlenstoffatomen steht, das gegebenenfalls durch Phenyl oder Naphthyl substituiert ist,
- $R^2$ für Wasserstoff oder für eine Gruppe der Formel $-OR^4$ steht,
  worin
- $R^4$ Wasserstoff, geradkettiges oder verzweigtes Alkyl mit bis zu 6 Kohlenstoffatomen, Benzyl oder Phenyl bedeutet,
- $R^3$ für Hydroxy oder geradkettiges oder verzweigtes Alkoxy mit bis zu 6 Kohlenstoffatomen steht, oder
  für eine Gruppe der Formel $-NHSO_2-R^5$ steht,
  worin
- $R^5$ geradkettiges oder verzweigtes Alkyl mit bis zu 6 Kohlenstoffatomen bedeutet, das gegebenenfalls durch Phenyl substituiert ist, das seinerseits durch geradkettiges oder verzweigtes Alkyl mit bis zu 6 Kohlenstoffatomen, Fluor, Chlor oder Brom substituiert sein kann,
  oder
  Phenyl bedeutet, das gegebenenfalls durch Fluor, Chlor, Brom oder durch geradkettiges oder verzweigtes Alkyl mit bis zu 4 Kohlenstoffatomen substituiert sein kann

und deren Salze.

Besonders bevorzugt sind Verbindungen der allgemeinen Formel (I),
in welcher

- A, B, D, E und L gleich oder verschieden sind und
  für Wasserstoff, Fluor, Chlor, Brom, geradkettiges oder verzweigtes Alkyl oder Alkoxy mit jeweils bis zu 6 Kohlenstoffatomen stehen,
- G für ein Schwefelatom oder für die Gruppe der Formel -N=CH- steht,
- $R^1$ für Cyclopentyl, Cyclohexyl oder Cycloheptyl steht, oder
  für geradkettiges oder verzweigtes Alkyl mit bis zu 6 Kohlenstoffatomen steht, das gegebenenfalls durch Phenyl substituiert ist,
- $R^2$ für Wasserstoff oder für eine Gruppe der Formel $-OR^4$ steht,
  worin
- $R^4$ Wasserstoff, geradkettiges oder verzweigtes Alkyl mit bis zu 4 Kohlenstoffatomen oder Phenyl bedeutet,
- $R^3$ für Hydroxy oder geradkettiges oder verzweigtes Alkoxy mit bis zu 4 Kohlenstoffatomen steht, oder
für eine Gruppe der Formel $-NHSO_2-R^5$ steht,
worin

$R^5$ geradkettiges oder verzweigtes Alkyl mit bis zu 4 Kohlenstoffatomen bedeutet, das gegebenenfalls durch Phenyl substituiert ist, das seinerseits durch geradkettiges oder verzweigtes Alkyl mit bis zu 4 Kohlenstoffatomen, Fluor oder Chlor substituiert sein kann,
oder
Phenyl bedeutet, das gegebenenfalls durch Fluor, Chlor oder Methyl substituiert sein kann

und deren Salze.

Ganz besonders bevorzugt sind Verbindungen, in denen der Rest $-CR^1R^2-COR^3$ in 3- oder 4-Position zum Heterocyclylmethoxyrest steht.

Darüber hinaus wurde ein Verfahren zur Herstellung der Verbindungen der Formel (I) gefunden, das dadurch gekennzeichnet ist, daß man

[A] Verbindungen der allgemeinen Formel (Ia),

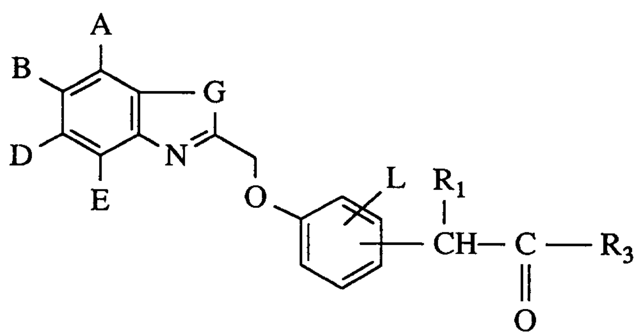

(Ia),

in welchen

A, B, D, E, G, L, $R^1$ und $R^3$ die angegebene Bedeutung haben,

werden hergestellt, indem man

Verbindungen der allgemeinen Formel (II)

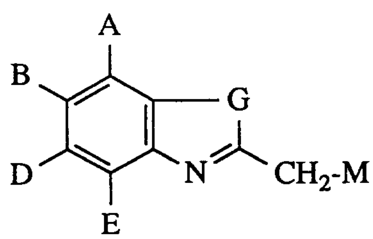

(II),

in welcher

A, B, D, E und G die angegebene Bedeutung haben

und

M für Halogen, vorzugsweise für Brom, steht,

entweder direkt mit Verbindungen der allgemeinen Formel (III)

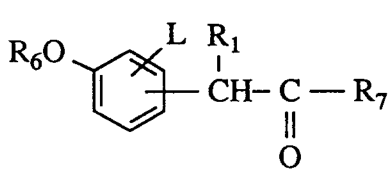

(III),

in welcher

$R^6$ für eine typische Hydroxyschutzgruppe wie beispielsweise Benzyl oder tert.-Butyl, vorzugsweise Benzyl, steht

und

$R^7$ für $C_1$-$C_8$-Alkoxy steht,

in inerten Lösemitteln, gegebenenfalls in Anwesenheit einer Base, nach Abspaltung der Schutzgruppe verethert,

oder

zunächst in einem ersten Schritt mit Verbindungen der allgemeinen Formel (IIIa)

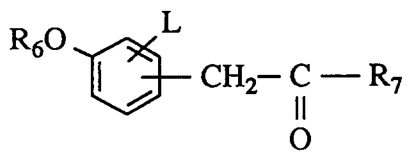

(IIIa),

in welcher

$R^6$ und $R^7$ die angegebene Bedeutung haben,

in inerten Lösemitteln, gegebenenfalls in Anwesenheit einer Base, nach Abspaltung der Schutzgruppe verethert und anschließend in einem zweiten Schritt mit Verbindungen der allgemeinen Formel (IV)

$R^1$-T (IV),

in welcher

$R^1$ die angegebene Bedeutung hat,

und

T für Halogen, vorzugsweise für Chlor oder Brom, steht,

in inerten Lösemitteln, gegebenenfalls in Anwesenheit einer Base alkyliert und

im Fall der Säuren ($R^3$ = OH) die Ester nach üblichen Methoden verseift,

und im Fall der Sulfonamide ($R^3$ = $NHSO_2R^5$), ausgehend von den entsprechenden Säuren, gegebenenfalls nach deren vorgeschalteter Aktivierung, durch Umsetzung mit den jeweiligen Sulfonaminen der allgemeinen Formel (VIII)

$H_2N$-$SO_2R^5$ (VIII)

in inerten Lösemitteln, gegebenenfalls in Anwesenheit einer Base und/oder Hilfsmittel, eine Amidierung anschließt,

und im Fall der verschiedenen Ester, gegebenenfalls eine Umesterung, ebenfalls ausgehend von den aktivierten Carbonsäuren, durchführt,

und im Fall der reinen Enantiomere die entsprechenden Säuren trennt,

und die Substituenten A, B, D, E und L gegebenenfalls nach üblichen Methoden einführt bzw. variiert,

[B] Verbindungen der allgemeinen Formel (IIb)

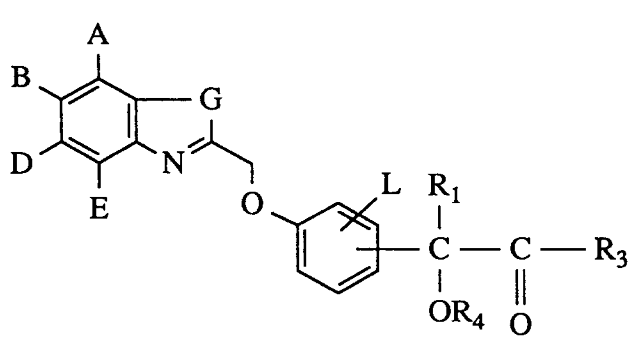

(IIb),

in welcher
A, B, D, E, G, L, $R^1$, $R^3$ und $R^4$ die angegebene Bedeutung haben,
werden hergestellt, indem man
Verbindungen der allgemeinen Formel (II) zunächst durch Umsetzung mit Verbindungen der allgemeinen Formel (V)

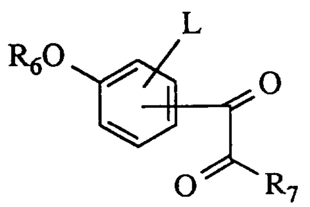

(V),

in welcher
L, $R^6$ und $R^7$ die oben angegebene Bedeutung haben,
in die Verbindungen der allgemeinen Formel (VI)

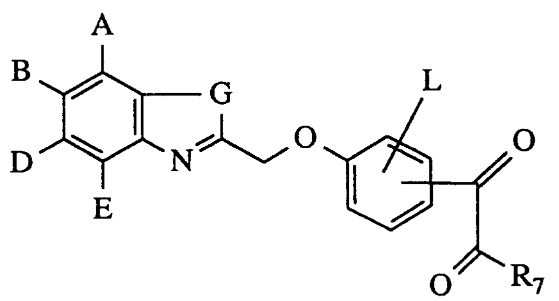

(VI),

in welcher
A, B, D, E, G, L und $R^7$ die oben angegebene Bedeutung haben,
durch Veretherung überführt
und in einem zweiten Schritt durch Umsetzung mit Grignard-Verbindungen der allgemeinen Formel (VII)

$R^1$-V (VII),

in welcher
$R^1$ die oben angegebene Bedeutung hat
und
V für den typischen Grignardrest W-Z steht,
worin
W Magnesium oder Zink bedeutet
und
Z Chlor, Brom oder Jod bedeutet,
oder
für Lithium, Natrium, Magnesium, Aluminium oder Zink steht,
in inerten Lösemitteln, gegebenenfalls in Anwesenheit einer Base, unter Abspaltung der Gruppe V reduziert,
und im Fall, daß $R^4 \neq H$, nach üblichen Methoden verethert
und
im Fall der Säuren ($R^3$ = OH) die Ester nach üblichen Methoden verseift,
und im Fall der Sulfonamide ($R^3$ = NH-$SO_2$-$R^5$), ausgehend von den entsprechenden Säuren, gegebenenfalls nach deren vorgeschalteter Aktivierung, durch Umsetzung mit den jeweiligen Sulfonaminen der allgemeinen Formel (VIII)

$H_2N$-$SO_2$-$R^5$ (VIII),

in welcher
$R^5$ die oben angegebene Bedeutung hat,
in inerten Lösemitteln, gegebenenfalls in Anwesenheit einer Base und/oder Hilfsmittels, eine Amidierung anschließt
und im Fall, der verschiedenen Ester, gegebenenfalls eine Umesterung, ebenfalls ausgehend von den aktivierten Carbonsäuren, durchführt,
und im Fall der reinen Enantiomere die entsprechenden enantiomerenreinen Säuren trennt,
und die Substituenten A, B, D, E und L gegebenenfalls nach üblichen Methoden einführt bzw. variiert.

Die erfindungsgemäßen Verfahren können durch folgendes Formelschema beispielhaft erläutert werden:

[A]

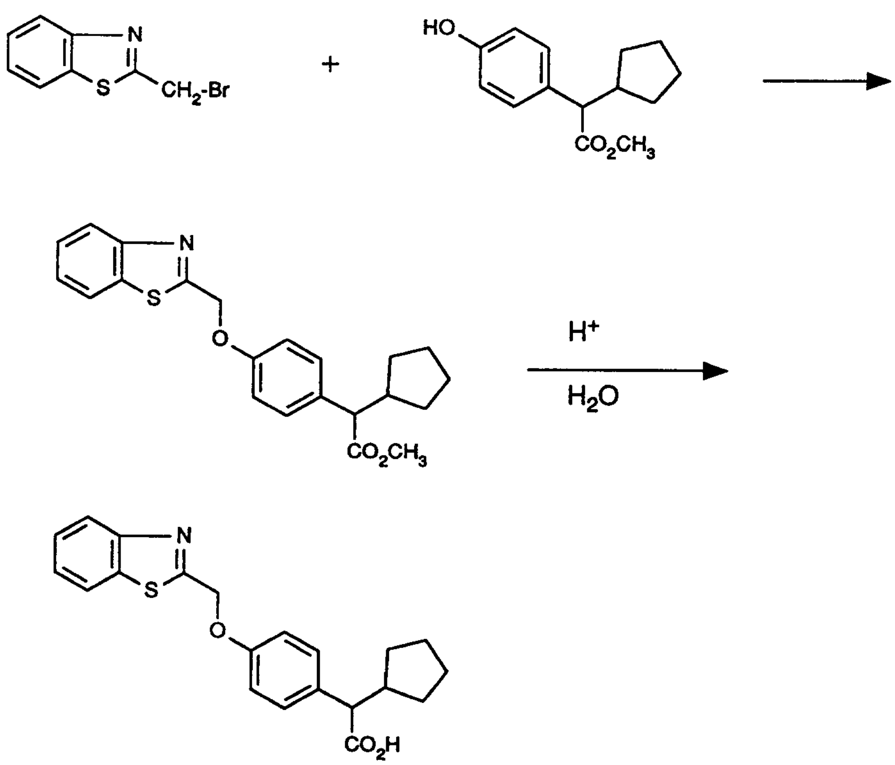

[A]

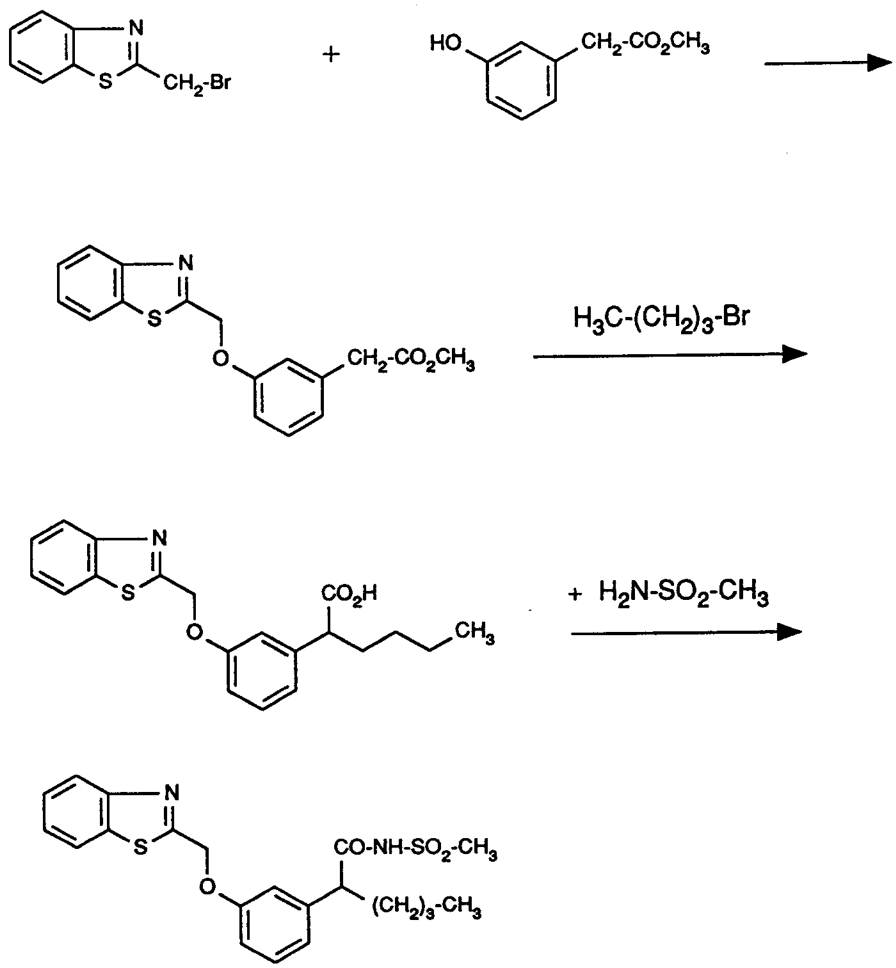

[B]

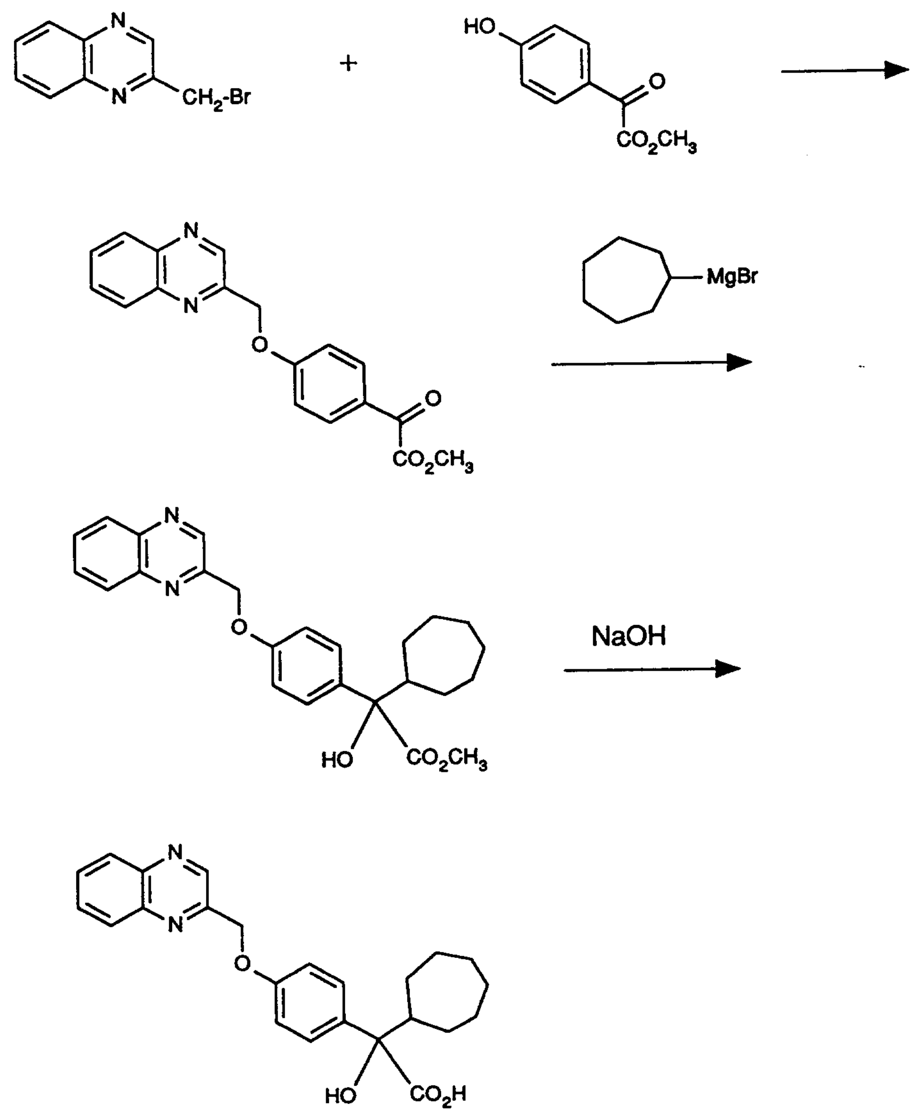


Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley / Sons, 1981, New York].

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhdyrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol des Reaktionspartners, ein. Die Base wird im allgemeinen in einer Menge von 0,5 bis 5 Mol, bevorzugt von 1 bis 3 Mol, bezogen auf das Halogenid, eingesetzt.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man molare Mengen der Reaktanden.

Als Lösemittel für die Reduktion eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenstoffe wie Benzol, Toluol, Xylol, Hexan, Cyclohexan, oder Erdölfraktionen oder Dimethylformamid. Ebenso ist es möglich, Gemische der genannten Lösemittel zu verwenden. Bevorzugt sind Tetrahydrofuran und Diethylether.

Die Reduktion wird im allgemeinen in einem Temperaturbereich von -80°C bis +30°C, bevorzugt bei -40°C bis +25°C, durchgeführt.

Die Reduktion wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Gruppe V erfolgt nach der für Grignard-Reaktionen üblichen Methode mit wäßriger Ammoniumchloridlösung [vgl. J. March, Advanced Organic Chemistry, Second Edition S. 836].

Im allgemeinen setzt man 1 bis 3 Mol, bevorzugt 1,1 Mol der Grignard Verbindungen bzw. der metallorganischen Verbindungen der allgemeinen Formel (VI), bezogen auf 1 Mol der Glyoxylester der allgemeinen Formel (V), ein.

Die Sulfamidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Sulfamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.butylat, oder organische Amine wie Benzyltrimethylammoniumhydroxid, Tetrabutylammoniumhydroxid, Pyridin, Triethylamin oder N-Methylpiperidin.

Die Sulfamidierung wird im allgemeinen in einem Temperaturbereich von 0°C bis 150°C, bevorzugt bei 25°C bis 40°C, durchgeführt.

Die Sulfamidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Sulfamidierung wird die Base im allgemeinen in einer Menge von 1 bis 3 Mol, bevorzugt von 1 bis 1,5 Mol, bezogen auf 1 Mol der Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Freeman et al., J. Biol. Chem. 225, 507 (1982) und N.B. Benoton, K. Kluroda, Int. Pept. Prot. Res. 13, 403 (1979), 17, 187 (1981)].

Die Verbindungen der allgemeinen Formel (II) sind größenteils bekannt oder können beispielsweise hergestellt werden, indem man die entsprechende Methylenfunktion in einem der oben aufgeführten Lösemittel, vorzugsweise Tetrachlorkohlenstoff, gegebenenfalls in Anwesenheit eines Katalysators, vorzugsweise AIBN mit Halogenierungsmitteln, vorzugsweise mit N-Bromsuccinimid, in einem Temperaturbereich von 20 °C bis 100 °C, vorzugsweise von 60 °C bis 80 °C und Normaldruck, halogeniert.

Die Verbindungen der allgemeinen Formeln (III), (IIIa) und (V) sind größenteils bekannt oder können nach üblicher Methode hergestellt werden [vgl. hierzu Chem. Commun. 1972, (11), 668].

Ebenso sind die Verbindungen der allgemeinen Formel (IV) bekannt [vgl. Beilstein 5.19 / 5.24 / 5.29] oder können nach üblichen Methoden aus den entsprechenden Alkoholen oder Cycloalkanen hergestellt werden.

Die Verbindungen der allgemeinen Formel (VI) sind neu und können nach dem oben angegebenen Verfahren hergestellt werden.

Die Verbindungen der allgemeinen Formel (VII) sind an sich bekannt oder können nach üblicher Methode hergestellt werden [vgl. K. Nützel, Houben-Weyl, Methoden der organischen Chemie, 4. Aufl. Bd. 13/2a, 53 ff. (Thieme Verlag, Stuttgart) 1973; M.S. Kharash, O. Reinmuth, Grignard Reactions of Nonmetallic Compounds, Prentice Hall, New York, 1974; Uhlman XII, 370; Houben-Weyl XIII/2a, 289-302; R.I. Trust, R.E. Ireland, Org. Synth. 53, 116, (1973); O. Grummitt, E.I. Becker, Org. Synth. Coll. Vol. IV, 771 (1963); H. Adkins, W. Zartman, Org. Synth. Coll. Vol. II, 606 (1943)].

Die Sulfonamine der allgemeinen Formel (VIII) sind bekannt oder können nach üblicher Methode hergestellt werden.

Die reinen Enantiomeren der erfindungsgemäßen Verbindungen der allgemeinen Formel (I) können beispielsweise hergestellt werden, indem man die entsprechenden enantiomerenreinen Säuren nach üblicher Methode trennt und anschließend wie oben aufgeführt weiter umsetzt.

Die Verbindungen der allgemeinen Formel (I) zeigen überraschenderweise eine hohe Aktivität als Leukotriensynthesehemmer insbesondere nach oraler Applikation.

Die erfindungsgemäßen Verbindungen können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Gicht, Thrombosen und Thromboembolien, Ischämien (periphere, cardiale, cerebrale Durchblutungsstörungen),Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen substituierten (Benzothiazolyl und Chinoxalin-2-yl-methoxy)phenyl-essigsäurederivate können sowohl in der Humanmedizin als auch in der Veterinärmedizin angewendet werden.

Die pharmakologischen Wirkdaten der erfindungsgemäßen Substanzen werden durch folgende Methode bestimmt:

Als Maß für die 5-Lipoxygenase-Hemmung (LOI) wurde die Freisetzung von Leukotrien $B_4$ ($LTB_4$) aus polymorphkernigen Humanleukozyten (PMN) nach Zugabe von Substanzen und $Ca^{++}$-Ionophor A 23187 mittels "reverse phase HPLC" nach Borgeat, P. et al., Proc. Nat. Acad. Sci. 76, 2148-2152 (1979), bestimmt.

| Bsp. Nr. | LOI $IC_{50}$ [mol/l] |
|---|---|
| 9 | $5,4 \times 10^{-6}$ |
| 10 | $2,8 \times 10^{-7}$ |
| 11 | $3,1 \times 10^{-7}$ |
| 15 | $1,8 \times 10^{-7}$ |
| 17 | $1,2 \times 10^{-7}$ |
| 18 | $2,8 \times 10^{-7}$ |
| 19 | $1,3 \times 10^{-7}$ |
| 20 | $4,4 \times 10^{-7}$ |
| 23 | $3,0 \times 10^{-7}$ |
| 24 | $2,4 \times 10^{-7}$ |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

4-Benzyloxyphenylessigsäuremethylester

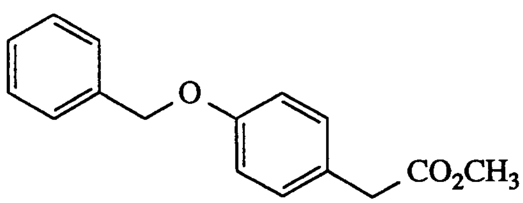

397 g (2,39 mol) 4-Hydroxyphenylessigsäuremethylester und 330 g (2,39 mol) Kaliumcarbonat werden in 2 l Dimethylformamid 1 h bei 25 °C gerührt. Danach werden 302 g (2,39 mol) Benzylchlorid zugegeben und 15 h auf 50 °C erwärmt. Nach Einengen im Vakuum wird der Rückstand zwischen Wasser und Essigester verteilt, die organische Phase über Natriumsulfat getrocknet und eingeengt. Das Produkt wird aus Methanol umkristallisiert.

Ausbeute: 511 g (83% der Theorie)

Festpunkt: 60 °C

In Analogie zur Vorschrift des Beispiels I werden die in Tabelle I aufgeführten Verbindungen hergestellt:

Tabelle I:

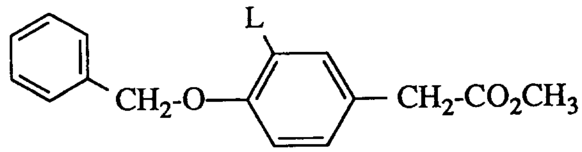

| Bsp.-Nr. | L | F°C | Ausbeute (% d.Th.) |
|---|---|---|---|
| II | -Cl | 72-74 | 98 |
| III | -Br | 40 | 91 |

Beispiel IV

2-(4-Benzyloxyphenyl)-2-cyclopentylessigsäuremethylester

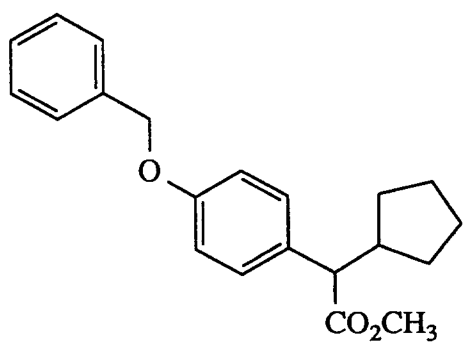

256,3 g (1 mol) der Verbindung aus Beispiel I werden in 1 l DMF gelöst und unter Schutzgas bei 0 °C in eine Suspension von 24 g (1 mol) Natriumhydrid in 100 ml DMF getropft. Nach beendeter $H_2$-Entwicklung wird 2 h bei 0 °C nachgerührt. Anschließend werden bei gleicher Temperatur 149 g (1 mol) Cyclopentylbromid, in 400 ml DMF gelöst, zugetropft. Nach beendeter Zugabe wird 15 h bei Raumtemperatur nachgerührt. Das Lösemittel wird abrotiert und der Rückstand mit heißem Wasser (80 °C) versetzt. Nach Rühren wird langsam abgekühlt Das kristallisierte Produkt wird abgesaugt gut mit Wasser gewaschen, getrocknet und aus Methanol umkristallisiert.
Ausbeute: 276 g (85% der Theorie)
Festpunkt: 77-78 °C (Methanol)
In Analogie zur Vorschrift des Beispiels IV werden die in Tabelle II aufgeführten Verbindungen hergestellt:

Tabelle II:

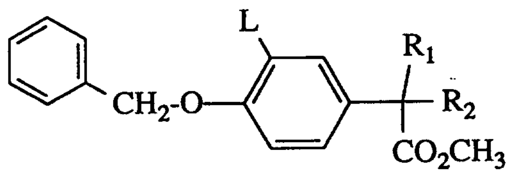

| Bsp.-Nr. | L | $R^1$ | $R^2$ | F°C / Kp. | Ausbeute (% d. Th.) |
|---|---|---|---|---|---|
| V | H | $-(CH_2)_3-CH_3$ | H | 145-150 0,2 mm | 87 |
| VI | H | 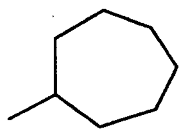 | H | 59 | 76 |
| VII | Cl | 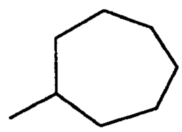 | H | 165-170 0,2 mm | 74 |

Beispiel VIII

2-Cyclopentyl-2-(4-hydroxyphenyl)essigsäuremethylester

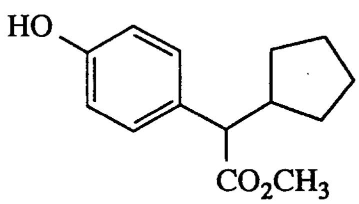

65 g (0,2 mol) der Verbindung aus Beispiel IV werden in 100 ml THF, 200 ml Ethanol und 100 ml Triethylamin gelöst, Nach Zugabe von 1,5 g Palladium-Katalysator (10%ig auf Kohle) wird 2 h bei 3 bar Wasserstoff hydriert. Der Katalysator wird abfiltriert, das Filtrat eingeengt und an Kieselgel (Eluens : Methylenchlorid) chromatographiert.
Ausbeute: 43,7 g (93% der Theorie)
kp.: 150-153 / 0,2 mm
In Analogie zur Vorschrift des Beispiels VIII werden die in Tabelle III aufgeführten Verbindungen hergestellt:

Tabelle III:

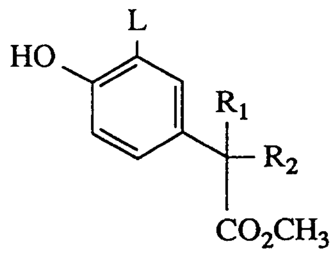

| Bsp.-Nr. | L | $R^1$ | $R^2$ | F°C / Kp. | Ausbeute (% d.Th.) |
|---|---|---|---|---|---|
| IX | H | $-(CH_2)_3-CH_3$ | H | 134-138 0,2 mm | 81 |
| X | H | 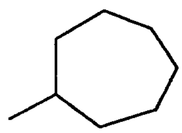 | H | 80 | 79 |
| XI | Cl | 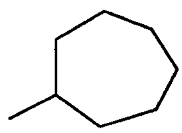 | H | 155-160 0,2 mm | 74 |

Beispiel XII

2-[3-(Benzothiazol-2-yl-methoxy)phenyl]essigsäuremethylester

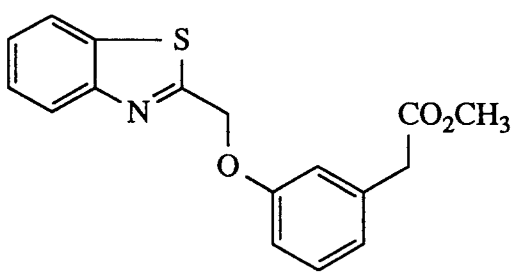

14,9 g (0,1 mol) 2-Methylbenzthiazol werden in 250 ml $CCl_4$ mit 19,6 g (0,11 mol) NBS und 0,5 g AIBN bromiert. Nach vollständiger Umsetzung wird das ausgefallene Succinimid abfiltriert, das Filtrat eingeengt, in 100 ml DMF aufgenommen und zu einer Lösung von 16,6 g (0,1 mol) 3-Hydroxyphenylessigsäuremethylester, 13,8 g (0,1 mol) $K_2CO_3$ in 250 ml DMF gegeben. Es wird 10 h auf 50 °C erwärmt. Nach Abkühlen wird das Lösemittel abgedampft, der Rückstand zwischen Essigester / Wasser verteilt, die organische Phase eingeengt und der Rückstand über Kieselgel 60 (Eluens: $CH_2Cl_2$) chromatographiert.
Ausbeute: 11,7 g (37,3% d. Th.)
Festpunkt: 58-61 °C (Diisopropylether)

Beispiel XIII

2-[3-Bromo-4-(chinoxalin-2-yl-methoxy)phenyl]essigsäuremethylester

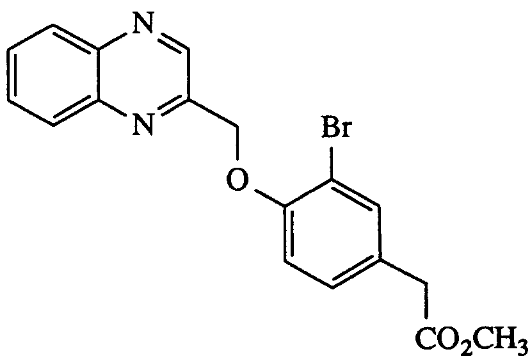

In Analogie zur Vorschrift des Beispiels XII wird die Titelverbindung hergestellt.
Ausbeute: 4,7 g (94,2% der Theorie)
Festpunkt: 120-122 °C (Essigester)

Beispiel XIV

4-(Chinoxalin-2-yl-methoxy)phenylglyoxylsäuremethylester

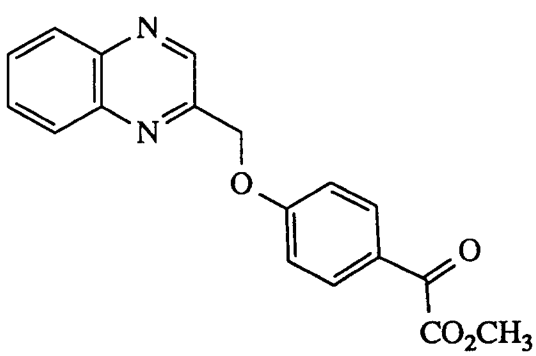

Die Titelverbindung wird in Analogie zur Vorschrift des Beispiels I hergestellt.
Ausbeute: 8,0 g (37,7% der Theorie)
Festpunkt: 125 °C (Methanol)

Beispiel XV

4-(Benzothiazol-2-yl-methoxy)phenylglyoxylsäuremethylester

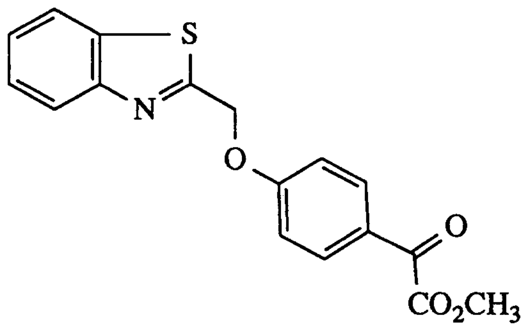

Die Titelverbindung wird in Analogie zur Vorschrift des Beispiels XIV hergestellt.
Ausbeute: 10,3 g (31,4% der Theorie)
Festpunkt: 114 °C (Methanol)

Herstellungsbeispiele

Beispiel 1

2-[4-(Benzothiazol-2-yl-methoxy)phenyl]-2-cyclopentylessigsäuremethylester

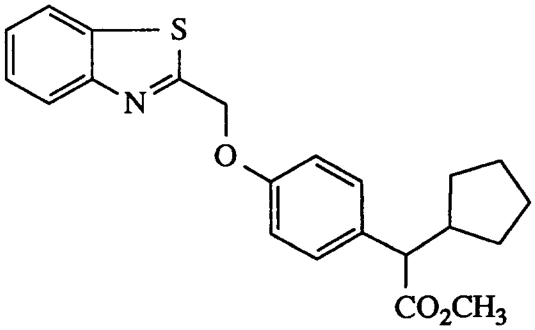

3,0 g (20 mmol) 2-Methylbenzothiazol werden in 50 ml $CCl_4$ mit 4,0 g (22,5 mmol) N-Brom-succinimid und 0,3g AIBN bromiert. Nach Umsetzung wird das ausgefallene Succinimid abfiltriert, das Filtrat eingeengt, in 50 ml DMF aufgenommen und zu einer Lösung von 4,7 g (20 mmol) 2-Cyclopentyl-2-(4-hydroxyphenyl)-essigsäuremethylester, 2,8 g (20 mmol) $K_2CO_3$ in 100 ml DMF gegeben. Es wird 10 h auf 50 °C erwärmt. Nach Abkühlen wird das Lösemittel abgedampft, der Rückstand zwischen Essigester/Wasser verteilt, die organische Phase eingeengt, und der Rückstand über Kieselgel 60 (Eluens: Cyclohexan/Essigester 3:1) chromatographiert.
Ausbeute: 1,7 g (22,3% der Theorie)
Festpunkt: 97-98 °C (Diisopropylether)
In Analogie zur Vorschrift des Beispiels 1 werden die in Tabelle 1 aufgeführten Verbindungen hergestellt:

Tabelle 1:

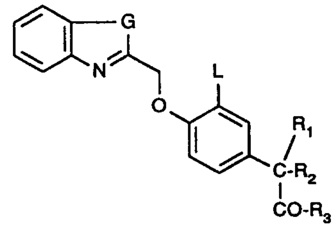

| Bsp.-Nr. | G | L | $R^2$ | $R^1$ | $R^3$ | F°C (Lösemittel) | Ausbeute g (% d.Th.) |
|---|---|---|---|---|---|---|---|
| 2 | S | H | H | $-(CH_2)_3CH_3$ | $-OCH_3$ | 65-66 (Diisopropyl-ether) | 2,6 (14,1) |
| 3 | -N=CH- | H | H | 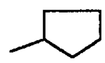 | $-OCH_3$ | 95-97 (Diisopropyl-ether) | 5,7 (68) |
| 4 | -N=CH- | Cl | H | 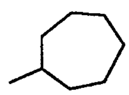 | $-OCH_3$ | 98-100 (Diisopropyl-ether) | 2,3 (90) |
| 5 | -N=CH- | H | H | 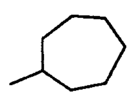 | $-OCH_3$ | 108-110 (Diisoproyl-ether) | 1,7 (93) |

Beispiel 6

2-[3-(Benzothiazol-2-yl-methoxy)phenyl]capronsäuremethylester

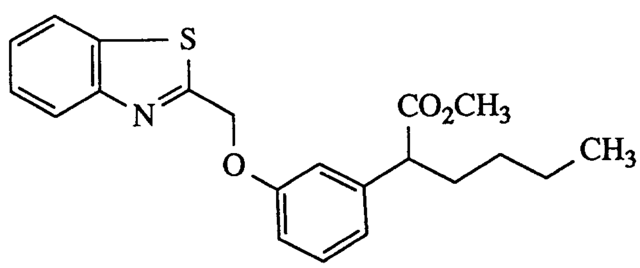

Die Titelverbindung wird in Analogie zur Vorschrift des Beispiels IV aus 10,9 g (0,035 mol) der Verbindung aus Beispiel IX hergestellt.
Ausbeute: 6,4 g (50% der Theorie)
Festpunkt: 108 ° C (Methanol)

Beispiel 7

2-[3-Bromo-4-(chinoxalin-2-yl-methoxy)phenyl]-2-cycloheptylessigsäuremethylester

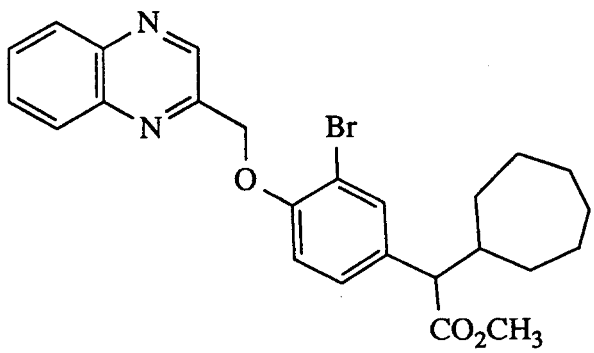

Die Titelverbindung wird in Analogie zur Vorschrift des Beispiels 6 hergestellt.
Ausbeute: 2,1 g (40% der Theorie)
Festpunkt: 132 °C (Methanol)

Beispiel 8

2-[4-(Benzothiazol-2-yl-methoxy)phenyl]-2-cyclopentylessigsäure

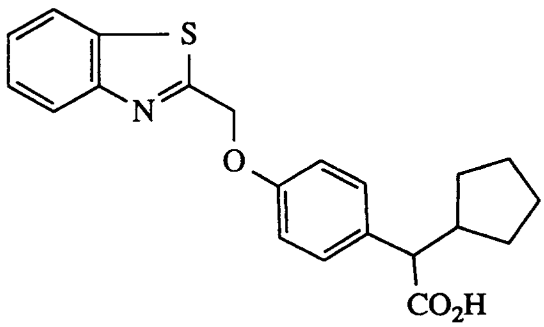

1,5 g (3,9 mmol) 2-[4-(Benzothiazol-2-yl-methoxy)phenyl]-2-cyclopentylessigsäuremethylester werden in 50 ml Methanol und 20 ml NaOH (1 normal) unter Rückfluß verseift. Nach Abkühlen werden 20 ml HCl (1 normal) zugegeben. Das Produkt fällt aus. Nach Absaugen wird mit Wasser gewaschen, getrocknet und aus Methanol umkristalliisert.
Ausbeute: 1,3 g (97% der Theorie)
Festpunkt: 152-154 °C (Methanol)
In Analogie zur Vorschrift des Beispiels 8 werden die in Tabelle 2 aufgeführten Verbindungen hergestellt:

Tabelle 2:

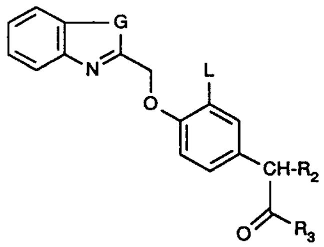

| Bsp.-Nr. | G | L | $R^2$ | $R^3$ | F°C (Lösemittel) | Ausbeute g (% d.Th.) |
|---|---|---|---|---|---|---|
| 9 | -N=CH- | H | 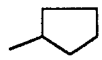 | OH | 173-175 (Diethyl-ether) | 3,2 (60) |
| 10 | -N=CH- | Cl | 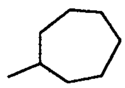 | OH | 101-103 (Diethyl-ether) | 1,7 (78,4) |
| 11 | -N=CH- | H | 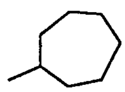 | OH | 163-165 ($CH_2Cl_2$) | 1,2 (87) |
| 12 | S | H | $-(CH_2)_3CH_3$ | OH | 117-118 (Methanol) | 2,1 (67,8) |
| 13 | -N=CH- | Br | 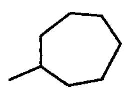 | OH | 149 ($CH_3OH$) | 1,3 (51,5) |
| 14 | S | H | $-(CH_2)_3-CH_3$ | OH | 99-102 ($CH_3OH$) | 1,9 (95) |

Beispiel 15

2-[3-(Benzothiazol-2-yl-methoxy)phenyl]-capronsäure-N-methylsulfonylamid

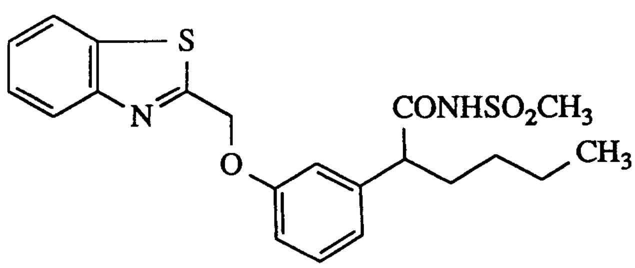

1 g (2,8 mmol) 2-[3-Benzothiazol-2-yl-methoxy)phenyl]-capronsäure, 0,267 g (2,8 mmol) Methansulfonamid, 0,54 g (2,8 mmol) N'-Ethyl-N'-(3-dimethylaminopropyl)carbodiimid und 0,45 g (3,7 mmol) DMAP werden in 30 ml $CH_2Cl_2$ gelöst und 15 h bei 25 °C gerührt. Das Lösemittel wird abgedampft und der Rückstand an Kieselgel 60 (Eluens: $CH_2Cl_2$ / MeOH 100:5) chromatographiert.
Ausbeute: 0,42g (34,7% der Theorie)

Festpunkt: 99-101 °C (Methanol)
In Analogie zur Vorschrift des Beispiels 15 werden die in Tabelle 3 aufgeführten Verbindungen hergestellt:

Tabelle 3:

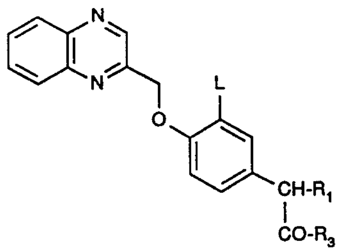

| Bsp.-Nr. | L | $R^1$ | $R^3$ | F°C (Lösemittel) | Ausbeute g (% d.Th.) |
|---|---|---|---|---|---|
| 16 | Cl | 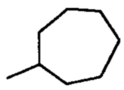 | $-NHSO_2CH_3$ | 163-165 (Diethyl-ether) | 0,3 (34) |
| 17 | Cl | 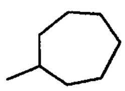 | $-NHSO_2-CH_2-C_6H_5$ | 150-152 (Diethyl-ether) | 0,7 (53,6) |
| 18 | H | 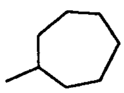 | $-NHSO_2CH_3$ | 160-162 (Diethyl-ether) | 0,5 (51,4) |
| 19 | H | 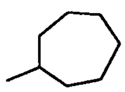 | $-NHSO_2-CH_2-C_6H_5$ | 148-150 (Diethyl-ether) | 0,6 (57) |
| 20 | H | 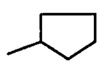 | $-NH-SO_2-(C_6H_4)-p-CH_3$ | 137 (Diethyl-ether) | 1,3 (92,5) |

Beispiel 21

2-[4-(Chinoxalin-2-yl-methoxy)phenyl]-2-cycloheptylhydroxyessigsäuremethylester

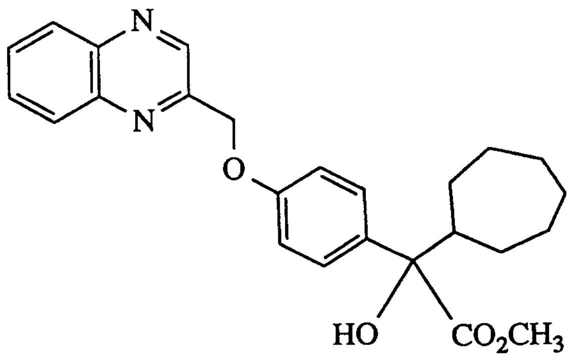

Zu einer gekühlten Lösung (0 °C) von 5 g (1,55 mmol) 2-[4-(Chinoxalin-2-yl-methoxy)phenyl]-glyoxylsäuremethylester in 50 ml THF wird unter Schutzgas eine frisch bereitete etherische Grignard-Lösung aus 5,49 g (3,1 mmol) Cycloheptylbromid und 0,75 g (3,1 mmol) Magnesium zugetropft. Nach Erwärmen auf 25 °C wird über Nacht gerührt, das Reaktionsgemisch auf Eiswasser gegossen, mit HCL angesäuert und mitEssigester extrahiert. Nach Eindampfen wird der Rückstand an Kieselgel 60 (Eluens: Cyclohexan / Essigester 3:1) chromatographiert.
Ausbeute: 2,26 g (34,7% der Theorie)
Festpunkt: 121 °C ($CH_3OH$)
In Analogie zur Vorschrift des Beispiels 21 werden die in Tabelle 4 aufgeführten Verbindungen hergestellt:

Tabelle 4:

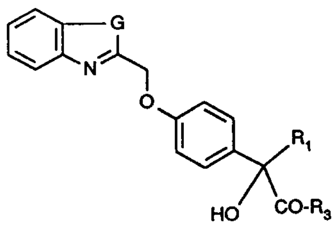

| Bsp.-Nr. | G | $R^1$ | $R^3$ | F°C (Lösemittel) | Ausbeute g (% d.Th.) |
|---|---|---|---|---|---|
| 22 | S | 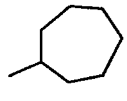 | $-OCH_3$ | 101 ($CH_3OH$) | 2,56 (39,4) |
| 23 | -N=CH- | 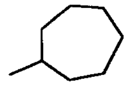 | -OH | 160 ($CH_3OH$) | 1,1 (50,7) |
| 24 | S | 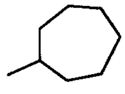 | -OH | 190 ($CH_3OH$) | 1,8 (86,5) |

## Patentansprüche

1. Substituierte (Benzothiazolyl- und Chinoxalyl-methoxy)phenyl-essigsäurederivate der allgemeinen Formel

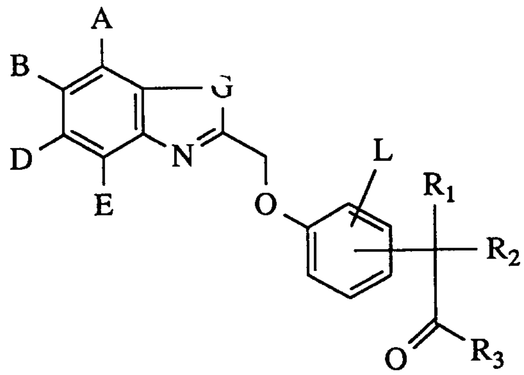

(I),

in welcher

| | |
|---|---|
| A, B, D, E und L | gleich oder verschieden sind und für Wasserstoff, Hydroxy, Halogen, Trifluormethyl, Trifluormethoxy oder Carboxy stehen, für geradkettiges oder verzweigtes Alkyl mit bis zu 10 Kohlenstoffatomen stehen, das gegebenenfalls durch Hydroxy oder Halogen substituiert ist, für geradkettiges oder verzweigtes Alkoxy oder Alkoxycarbonyl mit bis zu 10 Kohlenstoffatomen stehen, oder für Aryl mit 6 bis 10 Kohlenstoffatomen stehen, das gegebenenfalls durch Halogen, Nitro, Cyano oder durch geradkettiges oder verzweigtes Alkyl oder Alkoxy mit jeweils bis zu 8 Kohlenstoffatomen substituiert ist, |
| G | für ein Schwefelatom oder für die Gruppe der Formel -N = CH- steht, |
| $R^1$ | für Cycloalkyl mit 3 bis 12 Kohlenstoffatomen steht, oder für geradkettiges oder verzweigtes Alkyl mit bis zu 10 Kohlenstoffatomen steht, das gegebenenfalls durch Aryl mit 6 bis 10 Kohlenstoffatomen substituiert ist, |
| $R^2$ | für Wasserstoff, oder für eine Gruppe der Formel $-OR^4$ steht, worin |
| $R^4$ | Wasserstoff, geradkettiges oder verzweigtes Alkyl mit bis zu 8 Kohlenstoffatomen, Benzyl oder Phenyl bedeutet, |
| $R^3$ | für Hydroxy, geradkettiges oder verzweigtes Alkoxy mit bis zu 8 Kohlenstoffatomen oder Phenoxy steht, oder für eine Gruppe der Formel $-NHSO_2-R^5$ steht, worin |
| $R^5$ | geradkettiges oder verzweigtes Alkyl mit bis zu 8 Kohlenstoffatomen bedeutet, das gegebenenfalls durch Phenyl substituiert ist, das seinerseits durch geradkettiges oder verzweigtes Alkyl mit bis zu 6 Kohlenstoffatomen oder Halogen substituiert sein kann, oder Phenyl bedeutet, das gegebenenfalls durch Halogen, Trifluormethyl oder durch geradkettiges oder verzweigtes Alkyl oder Alkoxy mit jeweils bis zu 6 Kohlenstoffatomen substituiert sein kann |

und deren Salze.

2. Substituierte (Benzothiazolyl- und Chinoxalyl-methoxy)phenyl-essigsäurederivate nach Anspruch 1, wobei

| | |
|---|---|
| A, B, D, E und L | gleich oder verschieden sind und für Wasserstoff, Fluor, Chlor, Brom, Trifluormethoxy oder Carboxy stehen, für geradkettiges oder verzweigtes Alkyl mit bis zu 8 Kohlenstoffatomen stehen, das gegebenenfalls durch Hydroxy, Fluor, Chlor oder Brom substituiert ist, |

für geradkettiges oder verzweigtes Alkoxy oder Alkoxycarbonyl mit jeweils bis zu 8 Kohlenstoffatomen stehen, oder
für Phenyl stehen, das gegebenenfalls durch Fluor, Chlor, Brom, Nitro, Cyano oder durch geradkettiges oder verzweigtes Alkyl oder Alkoxy mit jeweils bis zu 6 Kohlenstoffatomen substituiert ist,

G für ein Schwefelatom oder für die Gruppe der Formel -N=CH- steht,

$R^1$ für Cyclopropyl, Cyclobutyl, Cyclopentyl, Cyclohexyl, Cycloheptyl oder Cyclooctyl steht, oder
für geradkettiges oder verzweigtes Alkyl mit bis zu 8 Kohlenstoffatomen steht, das gegebenenfalls durch Phenyl oder Naphthyl substituiert ist,

$R^2$ für Wasserstoff oder für eine Gruppe der Formel $-OR^4$ steht,
worin

$R^4$ Wasserstoff, geradkettiges oder verzweigtes Alkyl mit bis zu 6 Kohlenstoffatomen, Benzyl oder Phenyl bedeutet,

$R^3$ für Hydroxy oder geradkettiges oder verzweigtes Alkoxy mit bis zu 6 Kohlenstoffatomen steht, oder
für eine Gruppe der Formel $-NHSO_2-R^5$ steht,
worin

$R^5$ geradkettiges oder verzweigtes Alkyl mit bis zu 6 Kohlenstoffatomen bedeutet, das gegebenenfalls durch Phenyl substituiert ist, das seinerseits durch geradkettiges oder verzweigtes Alkyl mit bis zu 6 Kohlenstoffatomen, Fluor, Chlor oder Brom substituiert sein kann,
oder
Phenyl bedeutet, das gegebenenfalls durch Fluor, Chlor, Brom oder durch geradkettiges oder verzweigtes Alkyl mit bis zu 4 Kohlenstoffatomen substituiert sein kann

und deren Salze.

**3.** Substituierte (Benzothiazolyl- und Chinoxalyl-methoxy)phenyl-essigsäurederivate nach Anspruch 1, wobei

A, B, D, E und L gleich oder verschieden sind und
für Wasserstoff, Fluor, Chlor, Brom, geradkettiges oder verzweigtes Alkyl oder Alkoxy mit jeweils bis zu 6 Kohlenstoffatomen stehen,

G für ein Schwefelatom oder für die Gruppe der Formel -N=CH- steht,

$R^1$ für Cyclopentyl, Cyclohexyl oder Cycloheptyl steht, oder
für geradkettiges oder verzweigtes Alkyl mit bis zu 6 Kohlenstoffatomen steht, das gegebenenfalls durch Phenyl substituiert ist,

$R^2$ für Wasserstoff oder für eine Gruppe der Formel $-OR^4$ steht,
worin

$R^4$ Wasserstoff, geradkettiges oder verzweigtes Alkyl mit bis zu 4 Kohlenstoffatomen oder Phenyl bedeutet,

$R^3$ für Hydroxy oder geradkettiges oder verzweigtes Alkoxy mit bis zu 4 Kohlenstoffatomen steht, oder
für eine Gruppe der Formel $-NHSO_2-R^5$ steht,
worin

$R^5$ geradkettiges oder verzweigtes Alkyl mit bis zu 4 Kohlenstoffatomen bedeutet, das gegebenenfalls durch Phenyl substituiert ist, das seinerseits durch geradkettiges oder verzweigtes Alkyl mit bis zu 4 Kohlenstoffatomen, Fluor oder Chlor substituiert sein kann,
oder
Phenyl bedeutet, das gegebenenfalls durch Fluor, Chlor oder Methyl substituiert sein kann

und deren Salze.

**4.** Substituierte (Benzothiazolyl- und Chinoxalyl-methoxy)phenyl-essigsäurederivate nach Anspruch 1, wobei der Rest $-CR^1R^2-COR^3$ in 3- oder 4-Position zum Heterocyclylmethoxyrest steht.

**5.** Substituierte (Benzothiazolyl- und Chinoxalyl-methoxy)phenyl-essigsäurederivate zur therapeutischen Anwendung.

**6.** Verfahren zur Herstellung von substituierten (Benzothiazolyl- und Chinoxalyl-methoxy)-phenylessigsäurederivaten nach Anspruch 1, dadurch gekennzeichnet, daß man

[A] Verbindungen der allgemeinen Formel (Ia),

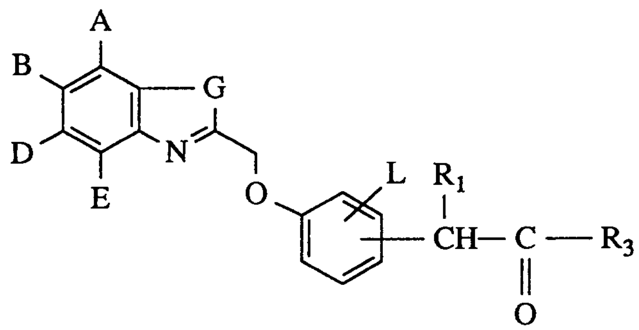

(Ia),

in welchen

A, B, D, E, G, L, $R^1$ und $R^3$ die angegebene Bedeutung haben,

werden hergestellt, indem man

Verbindungen der allgemeinen Formel (II)

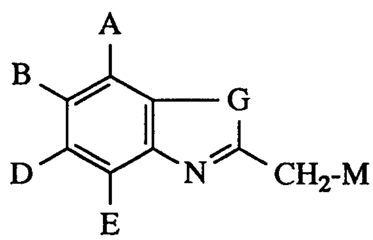

(II),

in welcher

A, B, D, E und G die angegebene Bedeutung haben

und

M für Halogen, vorzugsweise für Brom, steht,

entweder direkt mit Verbindungen der allgemeinen Formel (III)

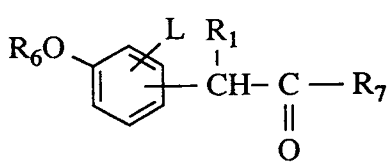

(III),

in welcher

$R^6$ für eine typische Hydroxyschutzgruppe wie beispielsweise Benzyl oder tert.-Butyl, vorzugsweise Benzyl, steht

und

$R^7$ für $C_1$-$C_8$-Alkoxy steht,

in inerten Lösemitteln, gegebenenfalls in Anwesenheit einer Base, nach Abspaltung der Schutzgruppe verethert,

oder

zunächst in einem ersten Schritt mit Verbindungen der allgemeinen Formel (IIIa)

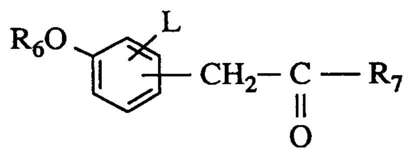

(IIIa),

in welcher

$R^6$ und $R^7$ die angegebene Bedeutung haben,

in inerten Lösemitteln, gegebenenfalls in Anwesenheit einer Base, nach Abspaltung der Schutzgruppe verethert und anschließend in einem zweiten Schritt mit Verbindungen der allgemeinen Formel (IV)

$R^1$-T (IV),

in welcher

$R^1$ die angegebene Bedeutung hat,

und

T für Halogen, vorzugsweise für Chlor oder Brom, steht,

in inerten Lösemitteln, gegebenenfalls in Anwesenheit einer Base alkyliert

und

im Fall der Säuren ($R^3$ = OH) die Ester nach üblichen Methoden verseift,

und im Fall der Sulfonamide ($R^3$ = $NHSO_2R^5$), ausgehend von den entsprechenden Säuren, gegebenenfalls nach deren vorgeschalteter Aktivierung, durch Umsetzung mit den jeweiligen Sulfonaminen der allgemeinen Formel (VIII)

$H_2N$-$SO_2R^5$ (VIII)

in inerten Lösemitteln, gegebenenfalls in Anwesenheit einer Base und/oder Hilfsmittel, eine Amidierung anschließt,

und im Fall der verschiedenen Ester, gegebenenfalls eine Umesterung, ebenfalls ausgehend von den aktivierten Carbonsäuren, durchführt,

und im Fall der reinen Enantiomere die entsprechenden Säuren trennt,

und die Substituenten A, B, D, E und L gegebenenfalls nach üblichen Methoden einführt bzw. variiert,

[B] Verbindungen der allgemeinen Formel (IIb)

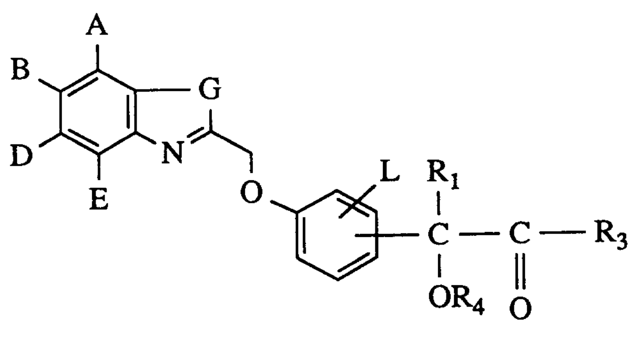

(IIb),

in welcher

A, B, D, E, G, L, $R^1$, $R^3$ und $R^4$ die angegebene Bedeutung haben,

werden hergestellt, indem man

Verbindungen der allgemeinen Formel (II) zunächst durch Umsetzung mit Verbindungen der allgemeinen Formel (V)

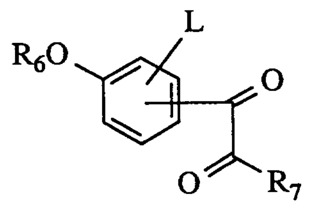

(V),

in welcher
L, $R^6$ und $R^7$ die oben angegebene Bedeutung haben,
in die Verbindungen der allgemeinen Formel (VI)

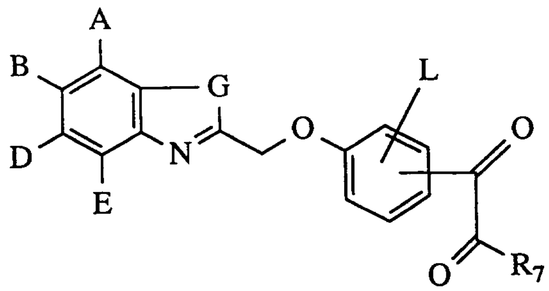

(VI),

in welcher
A, B, D, E, G, L und $R^7$ die oben angegebene Bedeutung haben,
durch Veretherung überführt
und in einem zweiten Schritt durch Umsetzung mit Grignard-Verbindungen der allgemeinen Formel (VII)

$R^1$-V (VII),

in welcher

$R^1$ die oben angegebene Bedeutung hat
und
V für den typischen Grignardrest W-Z steht,
worin
W Magnesium oder Zink bedeutet
und
Z Chlor, Brom oder Jod bedeutet,
oder
für Lithium, Natrium, Magnesium, Aluminium oder Zink steht,

in inerten Lösemittein, gegebenenfalls in Anwesenheit einer Base, unter Abspaltung der Gruppe V reduziert,
und im Fall, daß $R^4 \neq H$, nach üblichen Methoden verethert
und
im Fall der Säuren ($R^3$ = OH) die Ester nach üblichen Methoden verseift,
und im Fall der Sulfonamide ($R^3$ = NH-$SO_2$-$R^5$), ausgehend von den entsprechenden Säuren, gegebenenfalls nach deren vorgeschalteter Aktivierung, durch Umsetzung mit den jeweiligen Sulfonaminen der allgemeinen Formel (VIII)

$H_2N$-$SO_2$-$R^5$ (VIII),

in welcher
$R^5$ die oben angegebene Bedeutung hat,
in inerten Lösemitteln, gegebenenfalls in Anwesenheit einer Base und/oder Hilfsmittels, eine Amidierung anschließt
und im Fall, der verschiedenen Ester, gegebenenfalls eine Umesterung, ebenfalls ausgehend von den aktivierten Carbonsäuren, durchführt,
und im Fall der reinen Enantiomere die entsprechenden enantiomerenreinen Säuren trennt,
und die Substituenten A, B, D, E und L gegebenenfalls nach üblichen Methoden einführt bzw. variiert.

**7.** Arzneimittel enthaltend mindestens ein substituiertes (Benzothiazolyl- oder Chinoxalyl-methoxy)phenyl-essigsäurederivat nach Anspruch 1.

**8.** Verfahren zur Herstellung von Arzneimitteln nach Anspruch 6, dadurch gekennzeichnet, daß man die substituierte (Benzothiazolyl- und Chinoxalyl-methoxy)phenyl-essigsäurederivate gegebenenfalls mit Hilfe von üblichen Hilfs- und Trägerstoffen in eine geeignete Applikationsform überführt.

**9.** Verwendung von substituierten (Benzothiazolyl- und Chinoxalyl-methoxy)phenyl-essigsäurederivaten nach Anspruch 1 zur Herstellung von Arzneimitteln.

Europäisches Patentamt

# EUROPÄISCHER RECHERCHENBERICHT

Nummer der Anmeldung

EP 93 10 8996
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 219 436 (MITSUBISHI KASEI CORPORATION) * Beispiel 146, Zeile 71; Beispiel 182, Zeile 76 * --- | 1-9 | C07D277/64 C07D215/14 A61K31/425 A61K31/495 |
| Y | US-A-4 675 405 (MUSSER ET AL.) * das ganze Dokument * --- | 1-9 | |
| Y | US-A-4 594 425 (MUSSER ET AL.) * das ganze Dokument * --- | 1-9 | |
| Y | EP-A-0 385 680 (IMPERIAL CHEMICAL INDUSTRIES) * Beispiel 4(3) * --- | 1-9 | |
| Y | EP-A-0 181 568 (USV PHARMACEUTICAL CORPORATION) * Seite 55, Zeile 9 - Zeile 12 * --- | 1-9 | |
| Y,D | EP-A-0 339 416 (BAYER A.G.) * das ganze Dokument * --- | 1-9 | RECHERCHIERTE SACHGEBIETE (Int. Cl.5) |
| Y,D | EP-A-0 344 519 (BAYER A.G.) * das ganze Dokument * --- | 1-9 | C07D A61K |
| Y | EP-A-0 414 078 (BAYER A.G.) * das ganze Dokument * --- | 1-9 | |
| A | WO-A-9 106 537 (AMERICAN HOME PRODUCTS CORPORATION) * Beispiel 20 * --- | 1-9 | |
| A,P | EP-A-0 499 926 (BAYER A.G.) * Beispiele * --- | 1-9 | |
| A,P | EP-A-0 529 450 (BAYER A.G.) * Beispiele * --- -/-- | 1-9 | |

Der vorliegende Recherchenbericht wurde für alle Patentansprüche erstellt

| Recherchenort | Abschlußdatum der Recherche | Prüfer |
|---|---|---|
| BERLIN | 09 SEPTEMBER 1993 | FRELON D. L. M. G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 93 10 8996
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,P | EP-A-0 530 639 (BAYER A.G.)<br>* Beispiele *<br>----- | 1-9 | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| BERLIN | 09 SEPTEMBER 1993 | FRELON D. L. M. G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)